# EUROPEAN PATENT SPECIFICATION

(11) **EP 1 593 737 B1**
(45) Date of publication and mention of the grant of the patent: **02.02.2011**
(21) Application number: 04010723.7
(22) Date of filing: 05.05.2004
(51) Int. Cl.: C12N 5/07, C07K 14/47

(54) **Use of ACA glycoprotein antibodies for obtaining/maintaining pluripotent non-embryonic stem cells**
Verwendung von Glykoprotein ACA Antikörper zur Gewinnung oder Erhaltung von pluripotenten nicht-embryonalen Stammzellen
Utilisation d'anticorps contre la glycoprotéine ACA pour l'obtention ou le maintien de cellules souches pluripotentes non-embryonnaires

(43) Date of publication of application: 09.11.2005
(73) Proprietor: Becker-Kojic, Zorica, 69181 Leimen (DE)
(72) Inventor: Becker-Kojic, Zorica, 69181 Leimen (DE)

(56) References cited:
- EP-A- 1 391 463
- EP-A- 1 424 388
- WO-A-00/23567
- BECKER-KOJIC ZORICA ET AL: "A novel human erythrocyte glycosylphosphatidylinositol (GPI)-anchored glycoprotein ACA. Isolation, purification, primary structure determination, and molecular parameters of its lipid structure" JOURNAL OF BIOLOGICAL CHEMISTRY, AMERICAN SOCIETY OF BIOLOGICAL CHEMISTS, BALTIMORE, MD, US, vol. 277, no. 43, 25 October 2002 (2002-10-25), pages 40472-40478, XP002246776 ISSN: 0021-9258

## Description

The present invention relates to the use of a glycosylphosphatidylinositol(GPI)-anchored glycoprotein ACA or an activator of ACA for obtaining a pluripotent non-embryonic stem cell or for maintaining the pluripotential phenotype of non-embryonic stem cells.

Embryonic stem (ES) cells are the archetypical stem cells, being capable of differentiating to form the whole gamut of cell types found in the adult organism. Such stem cells are described as pluripotent cells as they are capable of differentiating into many cell types. Although ES cells have been isolated from human embryonic tissues, their use in research as well as therapeutics is encumbered by ethical considerations. Stem cells also exist for most tissues including haematopoietic, neural, gastrointestinal, epidermal, hepatic and mesenchymal stem cells. However, these stem cells have less self-renewal capacity and a more restricted capacity for differentiation, i.e. they are not pluripotent.

Until recently, it was thought that tissue-specific stem cells could only differentiate into cells of the tissue of origin. However, recent studies suggested that tissue-specific stem cells can differentiate into lineages other than the tissue of origin. After transplantation of bone marrow or enriched heamatopoietic stem cells, skeletal myoblasts, cardiac myoblasts, endothelium, hepatic and biliary duct epithelium, lung, gut and skin epithelia, and neuroectodermal cells of donor origin have been detected. Some studies demonstrated that neural stem cells as well as muscle cells may differentiate into haematopoietic cells. When injected into a blastocyst, neural stem cells contribute to a number of tissues of the chimeric mouse embryo. However, the studies carried out so far could not conclusively demonstrate that a tissue specific stem cell can differentiate into functional cells of multiple tissues, i.e. can be made pluripotent. To summarize, although there have been developed a pressing need to obtain non-embryonic (human) pluripotent stem cells (adult stem cells), so far the problems associated with stable long term culture for the propagation of such stem cells including the problem of lack of pluripotency have not been solved.

Thus, the technical problem underlying the present invention is to provide means allowing to obtain/maintain non-embryonic stem cells that are pluripotent.

The solution to the above technical problem has been achieved by providing the embodiments characterized in the claims. It has surprisingly been found that activation of ACA, a human GPI-anchored surface glycoprotein is sufficient to maintain the pluripotential phenotype of human hematopoietic stem cells. ACA is a 65 kD membrane glycoprotein with an unique phosphatidylinositol specific phospholipase C (PI-PLC) sensitive diacylglycerol structure of the lipid anchor isolated previously from human erythrocytes (Becker-Kojić and Terness, J. Biol. Chem. 2002; 277:40472-40478; EP-A1 1 391 463). In the studies resulting in the present invention rabbit polyclonal antibodies as well as mouse monoclonal antibodies to the purified protein were generated and used to analyse the expression of ACA protein on human peripheral blood cells by one and two-colour flow cytometry. The results showed that this protein is unimodally present on all, granulocytes, monocytes and B lymphocytes, but not on T-cells. Most importantly, a subset of CD34+/CD38- stem/progenitor cells in bone marrow also harbours ACA. It is apparent that in proliferating cells particularly those of malignant origin, ACA induces strong signals necessary for cell proliferation. This functional property is in line with the biochemical structure of the anchor which inserts ACA into the cell membrane. The glycerolipid derivatives of ACA resulting from hydrolysis with endogenous phospholipase C or D such as inositol glycan, diacylglycerol and phosphatidic acid are well known intermediates in membrane signalling events and activate the cascade of intracellular second messengers. Strong evidence is provided in the last years for lipid rafts, which are lipid based organizational platforms, containing GPI-linked proteins, tyrosine kinases and lipids in the membrane of living cells, playing a major role in many cellular processes. Cross-linking of some GPI-linked proteins induces up-regulation of activation associated cell surface proteins and production of growth-promoting cytokines.

The experiments illustrated below illuminate the putative novel ACA specific signal transduction pathway(s) involved in maintenance of self-renewal potential of human haematopoietic stem cells. Haematopoietic stem cells (=HSC) are characterized by the dual abilities to self renew and to differentiate into progenitors of multiple blood cells. These two feature require that HSC undergo asymmetric divisions to generate cells to sustained long haematopoiesis on the one hand, as well as the various progeny cells and distinct blood lineages on the other hand. CD34+/CD38- cells that divide asymmetrically gave rise to more blast colonies than those (CD34+/CD38+) which show symmetric division. Remarkably, following antibody induced ligation of ACA on the cell membrane, the progenitors cell population (CD34+/CD38+) turns to asymmetrically divided cells, clearly indicating a signalling competence of ACA protein. Mononuclear cells isolated from cord blood after ACA antibody ligation, give rise to manifold higher concentration of haematopoietic stem cells and thus indicate that this might be a novel method in order to get a stem cell population useful in clinical praxis.

### Brief description of the drawings

Figure 1:Representative analysis data of ACA expression on stem cells populations that were separated based on CD34+/CD38+ and CD34+/CD38- expression. (A) the isotype control, (B) ACA expression on CD34+/CD38+ and CD34+/ CD38- cells.
Figure 2: immunofluorescence images of CD34+/CD38+ cells stained with monoclonal antibody against human glycoprotein ACA.
Figure 3: immunofluorescence images of CD34+/CD38- cells stained with monoclonal antibody against ACA.
Figure 4: immunofluorescence images of CD34+/CD38+ cells before and 24 hours after cross-linking with anti-ACA specific antibody.

Thus, the present invention relates to the use of an anti-glycosylphosphatidylinositol(GPI)-anchored glycoprotein ACA antibody for obtaining pluripotent non-embryonic stem cells, for maintaining the pluripotenial phenotype of non-embryonic stem cells or for expanding pluripotent non-embryonic stem cells *ex vivo* such that differentiation of said stem cells is minimized or prevented.

As used herein, the term " non-embryonic stem cell" comprises any non-embryonic stem cell, e.g. a gonadal stem cell or somatic stem/progenitor cell, such as haematopoietic stem cell, epidermal stem cell, mesenchymal stem cell or neuronal stem cell with a stem cell derived from the haematopoietic system being preferred. Sources for obtaining and/or propagating pluripotent versions of said stem cells are e.g. bone marrow, peripheral blood, cord blood, fat tissue, liver, muscle, skeletal myoblasts, cardiac myoblasts, endothelium and epithelium.

As used herein, the term "a glycosylphosphatidylinositol(GPI)-anchored glycoprotein ACA" refers to the natural protein described in Becker-Kojić and Terness, 2002 and EP-A1 1 391 463

As used herein, the term "that differentiation of said stem cells is minimized or prevented" refers to culturing conditions wherein the stem cells substantially maintain the status of potency which they had before expansion.

The stem cells can be contacted with a glycosylphosphatidylinositol(GPI)-anchored glycoprotein ACA by several methods, e.g. (a) culturing the cells in a medium containing said protein, (b) transfecting the cells with a vector intracellularly expressing said protein in a secretable form, or (c) co-culturing the cells with producer cells expressing said fusion protein in a secretable form. The person skilled in the art knows suitable methods for transfection and suitable vectors; see e.g. DE 196 08 813 C2. The person skilled in the art can decide which approach is the most suitable one.

The person skilled in the art also knows suitable methods for culturing the stem cells and suitable media. The basal medium containing a protein of the invention can be any medium which is generally used for cultivation of mammalian (human) cells; see, e.g., DE 196 08 813 C2, EP-B1 0 695 351. In a preferred embodiment, the basal medium is IMEM (Life Technologies, Karlsruhe, Germany) or Cellgro (Cellgenix, Freiburg, Germany). The person skilled in the art can determine the optimum concentration of a glycosylphosphatidylinositol(GPI)-anchored glycoprotein ACA by simple experimentation; see also DE 196 08 813 C2. Preferably, the concentration of a glycosylphosphatidylinositol(GPI)-anchored glycoprotein ACA is in the range of 5 to 100 ng/ml medium.

The GPI-ACA used as additive to a medium can be prepared according to standard methods. Preferably, the protein is recombinantly produced or purified from natural sources as, e.g., described in Becker-Kojić and Terness, 2002. The term "antibody", preferably, relates to antibodies which consist essentially of pooled monoclonal antibodies with different epitopic specificities, as well as distinct monoclonal antibody preparations. As used herein, the term "antibody" (Ab) or "monoclonal antibody" (Mab) is meant to include intact molecules as well as antibody fragments (such as, for example, Fab and F(ab')2 fragments) which are capable of specifically binding to ACA. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, anti-ACA-antibodies include chimeric, single chain, and humanized antibodies

GPI-ACA may be used directly, or as an additive to a medium. It can also be supplied to the stem cells by intracellular expression and subsequent secretion. For intracellular expression any vector capable of replicating in mammalian cells, preferably an expression vector, can be used. Preferably, the DNA sequence encoding ACA is operatively linked to a suitable promoter. Suitable (expression) vectors are known to the person skilled in the art. Preferred recombinant vectors are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Furthermore, lenti viruses, such as HIV or SIV, are also preferred.

The construction of the recombinant vectors can be carried out according to conventional methods (cf. Sambrook et al., 1989, Molecular Cloning: A Laboratory Manual, 2nd edition, Cold Spring Harbor Laboratory Press, NY, USA). These vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts for amplifying said vectors include, but are not limited to, bacterial cells, such as E. coli, Streptomyces and Salmonella typhimurium cells, fungal cells, such as yeast cells, insect cells such as Drosophila S2 and Spodoptera Sf9 cells, animal cells, and plant cells.

Introduction of the above described vectors into the CD34+/CD38+ haematopoietic stem cell can be effected by well known methods, e.g. calcium phosphate transfection, DEAE-dextran mediated transfection, cationic lipid-mediated transfection, electroporation, nucleofection, transduction, infection, or other methods. Such methods are described in many standard laboratory manuals.

Haematopoietic stem cells (HSC) are defined as cells with the abilities of unrestricted self-renewal as well as multilineage differentiation into progenitor of all mature blood cell lineages. This discovery marked the beginning of modern day stem cell research. It has been suggested that a hallmark of a stem cell might be its ability to divide asymmetrically to produce a daughter cell identical to the mother and another cell committed to differentiation (Yeh and Lily, 1998, Nature 392:775). These two features require that the HCS undergo rounds of asymmetric divisions to generate mature cells of the distinct blood lineages as well as cells to sustain long term hematopoiesis. A central question in developmental biology is how hematopoietic stem cells divide in a self-renewing, asymmetric fashion, and how a single cell can divide to produce two daughter cells that adopt distinct fate. It is possible that two daughter cells from a HSC may be initially equivalent, but subsequent cell divisions must result in different fates of the progeny cells. The daughter cells might became different upon subsequent exposure to environmental signal, i.e., due to extrinsic factors. In the experiments described below is has been found that the proliferative potential for self-renew capacity of hematopoietic stem cell can be distributed through transfer from ACA protein from mother stem cell having contact to the one daughter.

The CD34+/CD38+ haematopoietic stem cells are HSC, preferably human HSC.

The following Examples illustrate the invention.

### Example 1: Analysis of ACA expression in various cell lines

### (A) Introduction

Lipid domains have unique functions in sorting membrane proteins and lipids and delivering them to specific intracellular sites. Biochemical studies, involving cold nonionic detergent extraction followed by sucrose density gradient flotation, show that kinases and many other signalling proteins are concentrated in a low density detergent-insoluble membrane fraction that is enriched in glycolipids and cholesterol. These structures have been called by many names, including detergent resistant membrane (DRMs), detergent-insoluble glycolipid-enriched domains (DIGs). They are believed to be derived from lipid rafts in the plasma membrane: cholesterol- and sphingolipid-enriched domains with lower fluidity than the bulk membrane forming so-called liquid-ordered phase (Simons and Ikonen, 1996, Nature 387:569-572).

These structure form spontaneously because the long saturated hydrophobic fatty acid tails of sphingolipids tend to associate together and pack with cholesterol. Phospholipids with saturated acyl chains also tend to partition into rafts, but in most cells the majority of plasma membrane phospholipids have unsaturated acyl groups and form a more fluid bulk phase. These structures are highly dynamic, because lipids are their main structural components and they have an intrinsically fast rate of lateral movement in the bilayer. Their dynamic behaviour makes them ideally suited to responding rapidly to changes in cell physiology. Many transmembrane proteins are excluded from lipid rafts although some partition into them; the reasons for these differences are largely unknown. However, the proteins most commonly found in lipid rafts carry one or more lipid modifications, such as acylation or glycosylphosphatidylinositol (GPI) anchors; the saturated acyl groups favour packing into liquid-ordered phase. Many of these proteins are involved in signalling and hence lipid rafts are frequently used by cells as supramolecular platforms for coordinating and enhancing signalling events.

Signal transduction pathways are modular composites of functionally interdependent sets of proteins that act in a coordinated fashion to transform environmental information into a phenotypic response. Many surface glycoproteins anchored in a membrane via glycosylphosphatidyl (GPI) moiety are unique in that they penetrate only the outer leaflet of the plasma membrane but are still able to mediate intracellular signalling events following antibody-induced ligation. They participate in the regulation of cell growth and differentiation and have been shown to be noncovalently associated with protein tyrosine kinases, key regulators of cell activation and signal transduction (Stefanova et al., 1991, Science 254:1016-1019). These so-called type III membrane proteins are therefore, devoid of any intracellular domain that could link them to cytoplasmic signal transuding molecules. Binding of natural ligands or antibodies to some GPI-linked proteins induced cell activation. Some of the biological activities of GPI molecules may be regulated by proteolytic or lypolytic cleavage products. These metabolites provide a potentially abundant source of pharmacoactive second messengers, including inositolphosphoglycans, inositolpeptidoglycans and diglycerides.

Currently, the phenotype of human HSCs and progenitors is not completely defined. Most studies suggest that human HCSs are small quiescent cells that express surface glycoprotein CD34. Data from many laboratory and clinical investigations indicate that CD34+ cells comprise approximately 1% of human bone marrow (BM) mononuclear cells including the progenitor cells of all the lymphohaematopoietic lineages and lymphohematopoietic stem cells, yet they generate the entire repertoire of the lymphhematopoietic system and they are the normal counterparts of the abnormal cells in myeloid leukemias, myelodisplasias, and aplastic anemias. Because stem cells are an important but rare cell type in the CD34+ cell population, investigators have subdivided CD34+ cell population to further enrich stem cells. The CD34+/CD38- subset comprises less than 10% of human CD34+ adult BM cells (<0.1% of marrow mononuclear cells), lacks expression of lineage commitment markers (*lin*)*,* contains cells with in vitro replating capacity, and is predicted to be highly enriched for stem cells. These cells purified from marrow using immunomagnetic microspheres or fluorescence-activated cell sorting generated easily detectable, long term, multilineage human hematopoiesis in the foetal sheep in vivo model. In contrast, transfer of CD34+/CD38+ cells to preimmune foetal sheep generated only short term human hematopoiesis suggesting that the CD34+/CD38+ cell population contains relatively early multipotent hematopoietic progenitor cells but not stem cells. (Curt et al., 1996, Blood 88:4102-4109). In summary, the CD34+/CD38- cell population has a high capacity for long term multilineage hematopietic engraftments, thus it is predicted that this cell population is highly enriched for stem cells.

The aim of the present experiments was to elucidate expression and functional significance of the human glycoprotein ACA in human hematopoietic stem cells.

### (B) Results

### Materials and Methods

Mononuclear blood cells isolated from umbilical cord blood and mobilised peripheral blood were enriched for CD34+ cells using immunomagnetic beads, or fluorescence-activated cell sorting, and ACA expression was investigated using anti-ACA specific monoclonal antibodies.

### (1) FACS analysis of ACA expression on human hematopoietic stem/progenitor cells

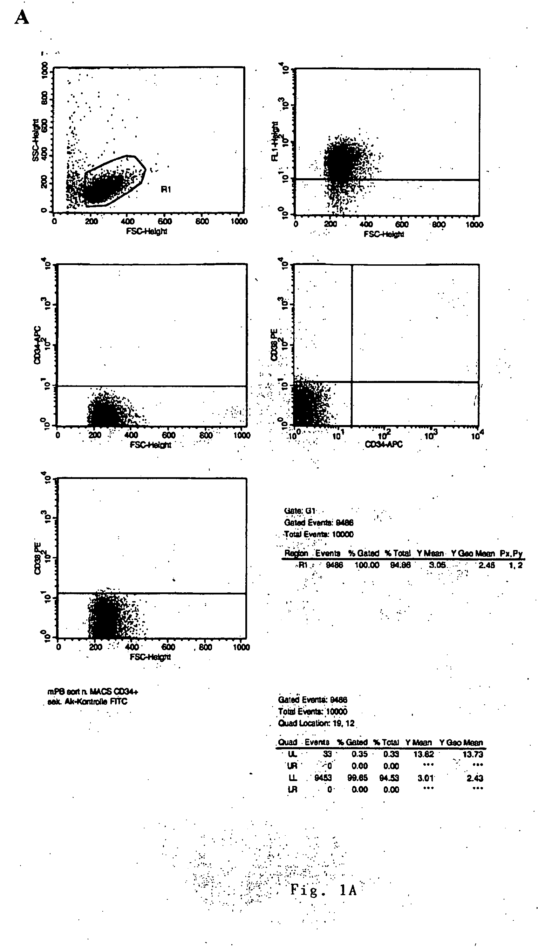

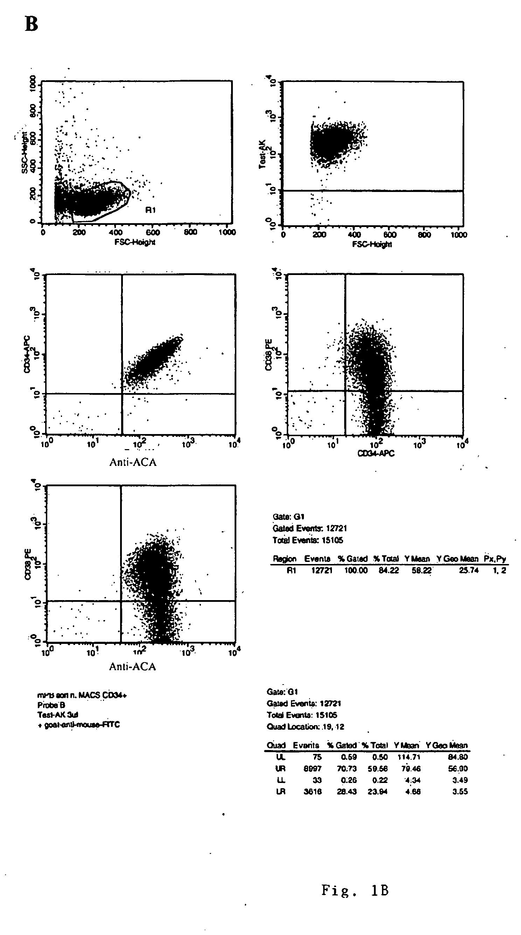

Mononuclear cells were isolated from mobilised peripheral blood cells(P. Mollee et al., Bone Marrow Transplant 2002; 30 273-278), stained with CD34 APC labelled antibody and CD34+ cells isolated in fluorescence-activated cell sorter. Enriched CD34+ cells were stained with CD38PE and ACA specific antibody and goat anti-mouse Alexa Fluor 488 (Mo Bi Tec GmbH, Göttingen, Germany) as second antibody, and analysed in fluorescence activated cell-sorter (Figure 1).

### (2) ACA expression on CD34+/CD38+ cells (human progenitor cell population)

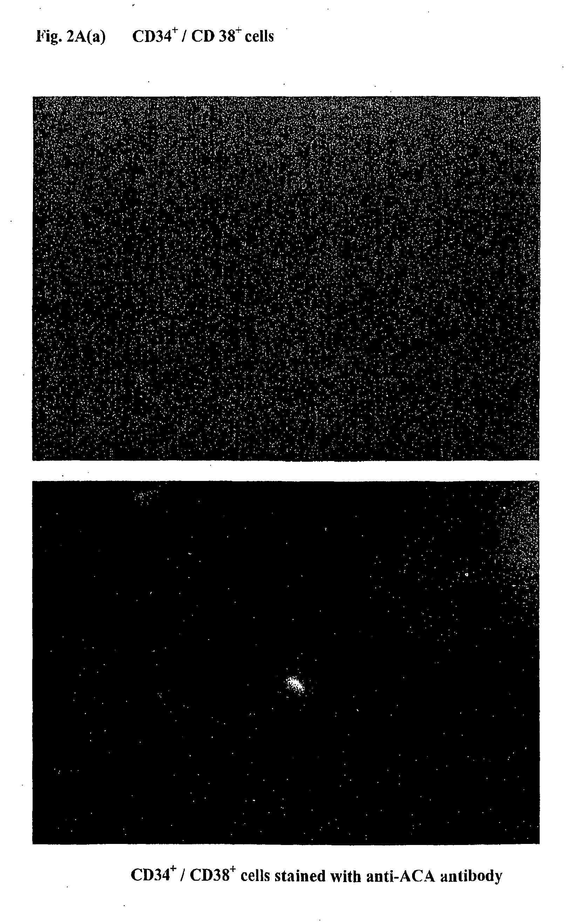

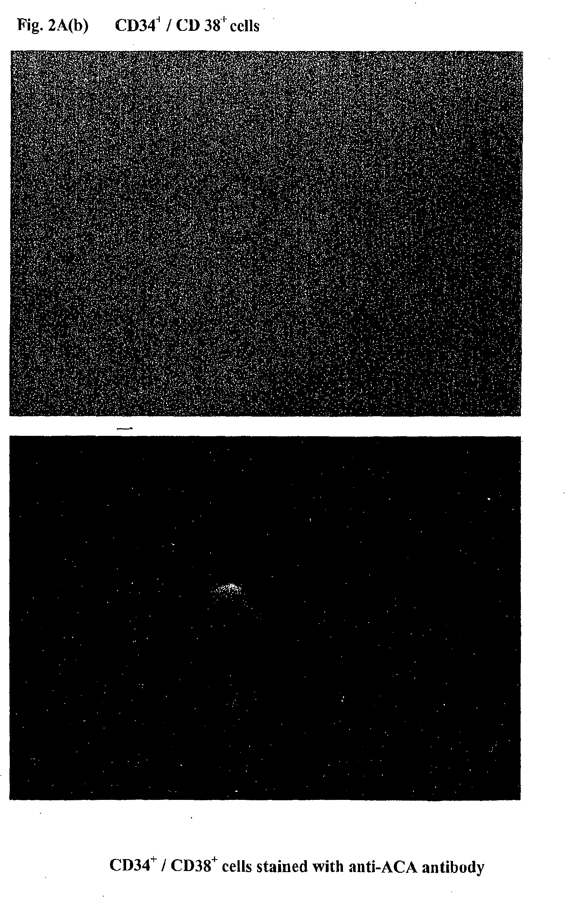

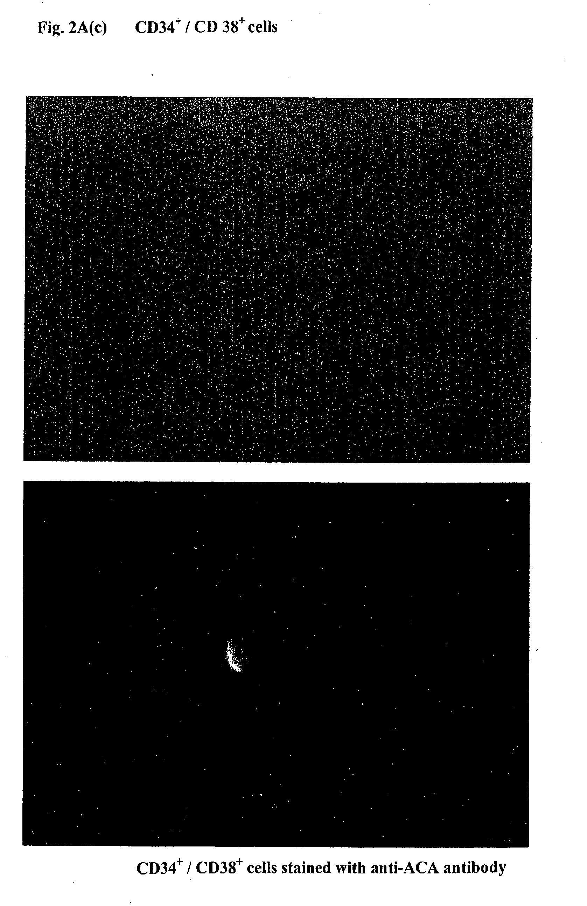

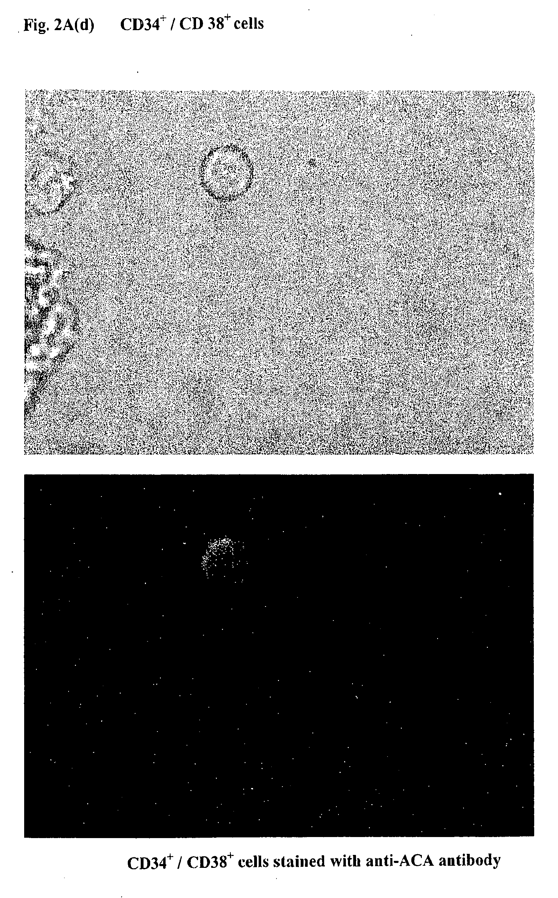

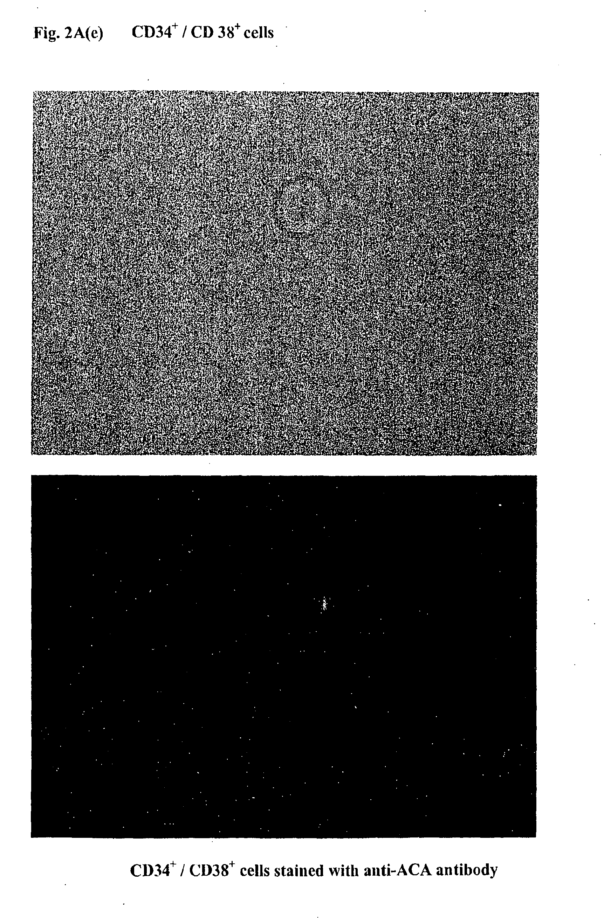

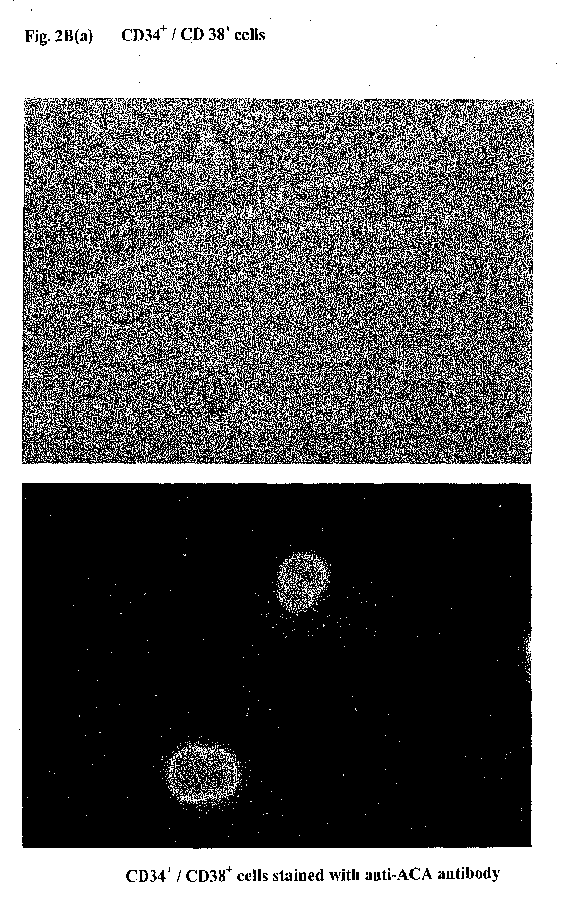

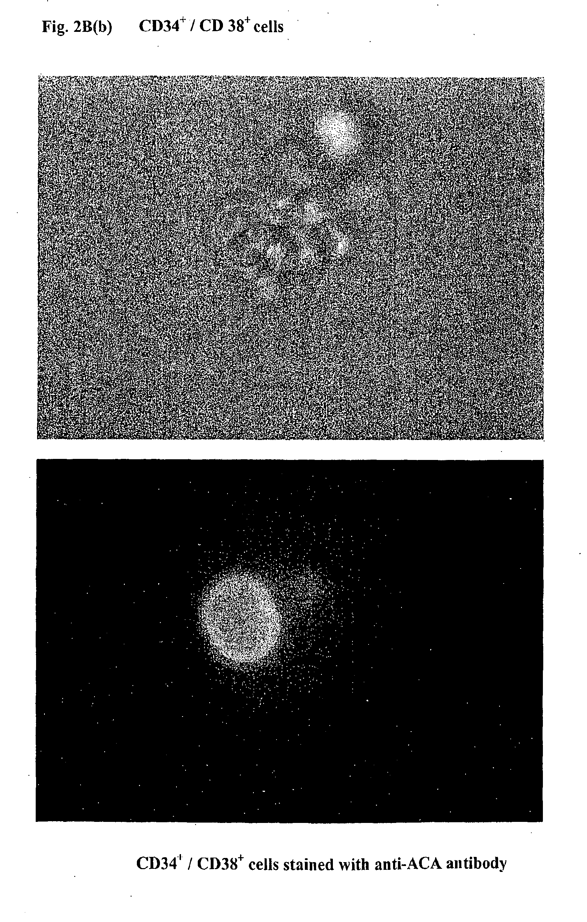

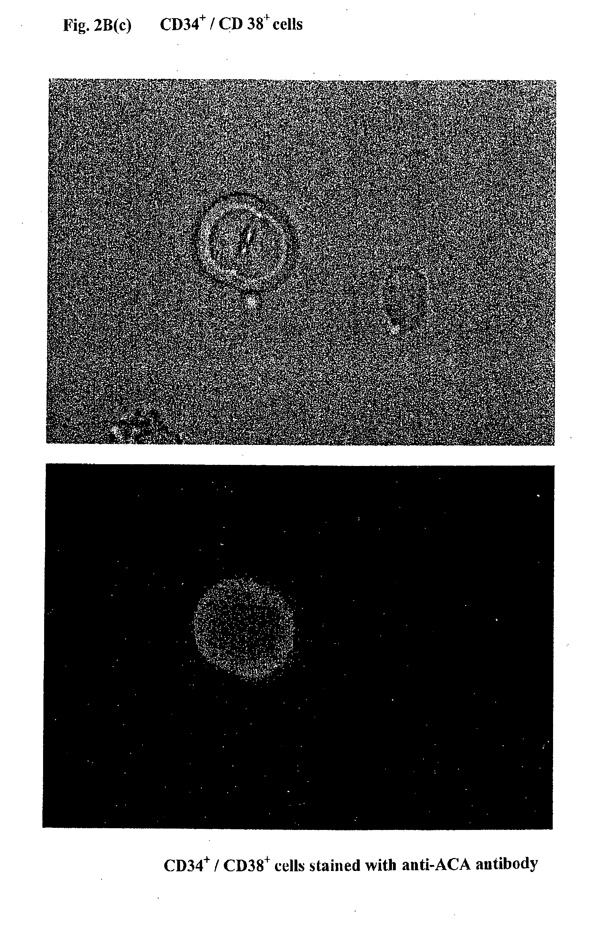

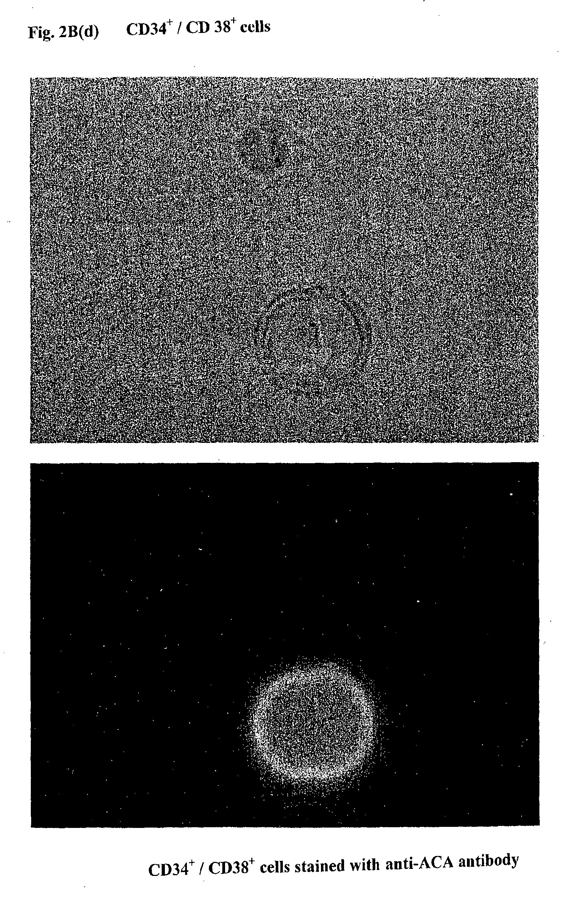

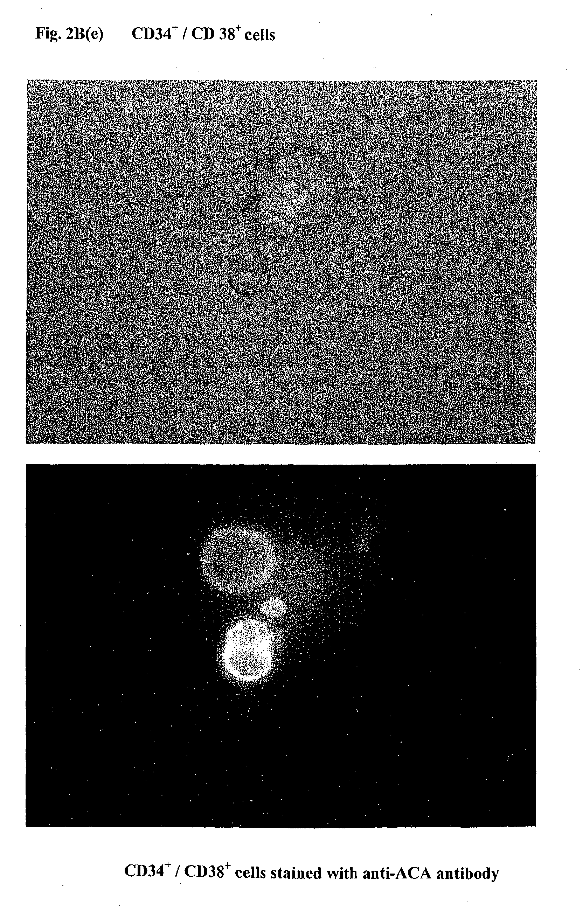

Mononuclear cells were isolated from umbilical cord blood, incubated with CD34 APC labelled antibody and CD34 positive cells magnetically sorted using MACS Anti-APC Micro Beads as described by supplier (Miltenyi Biotec, Germany). After staining with CD38 PE antibody CD34+/ CD38+ cells were purified using fluorescence activated cell-sorter. The isolated cells were placed in culture dishes with RPMI culture medium containing 10% FBS, incubated with ACA specific antibody ( EP 1 391 463 A1) and goat anti-mouse Alexa Fluor 488 as second antibody, and images were acquired using an inverted fluorescence microscope (Figure 2A). Mitotic stem /progenitor cells were cultured for 24h as describe above and then fix for five minutes at room temperature in 4% paraformaldehyde and after washing, incubated with anti-ACA antibody labelled with goat anti-mouse Alexa Fluor 488. Images were acquired using inverted fluorescence microscope. Mitotic stem/progenitor cells show the highest extent of ACA expression (Figure 2B).

### (3) ACA expression on CD34+/CD38- cells (human stem cell population)

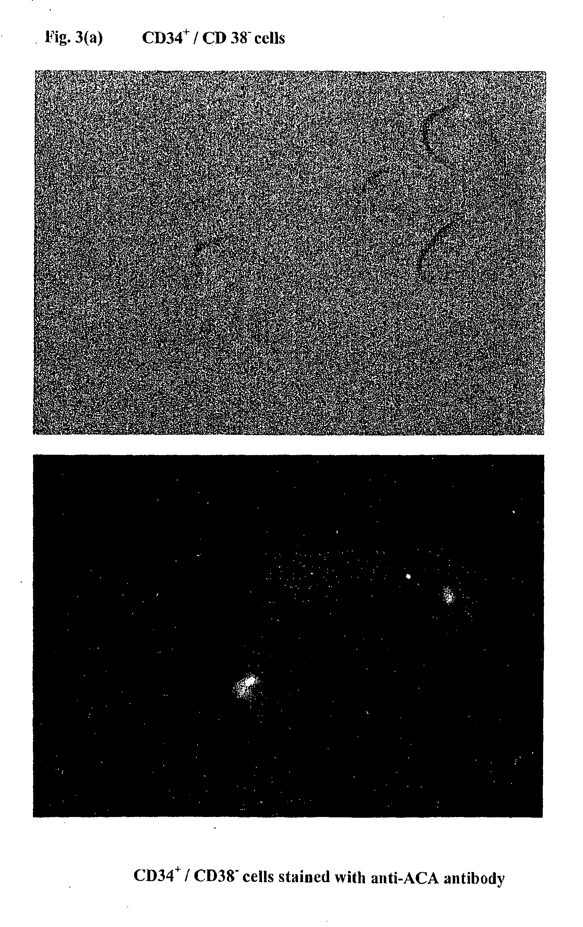

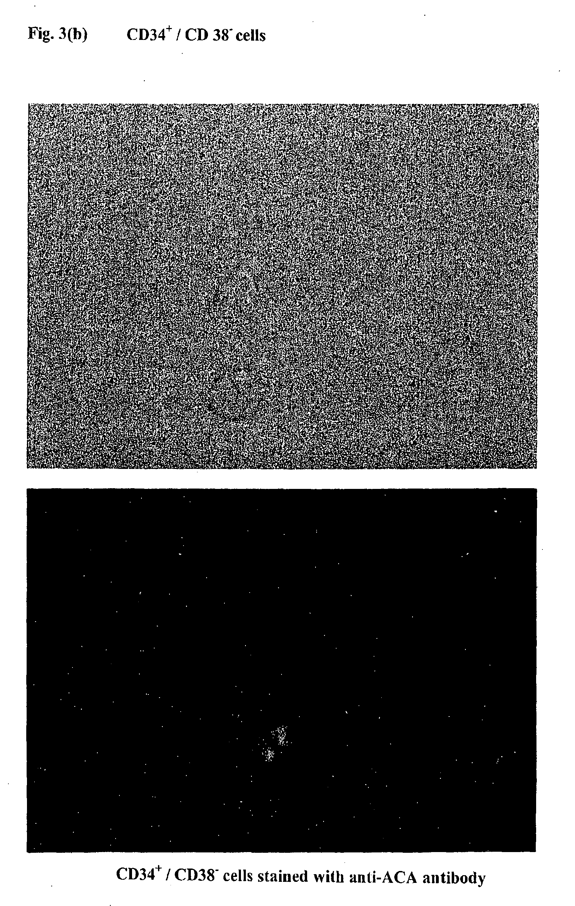

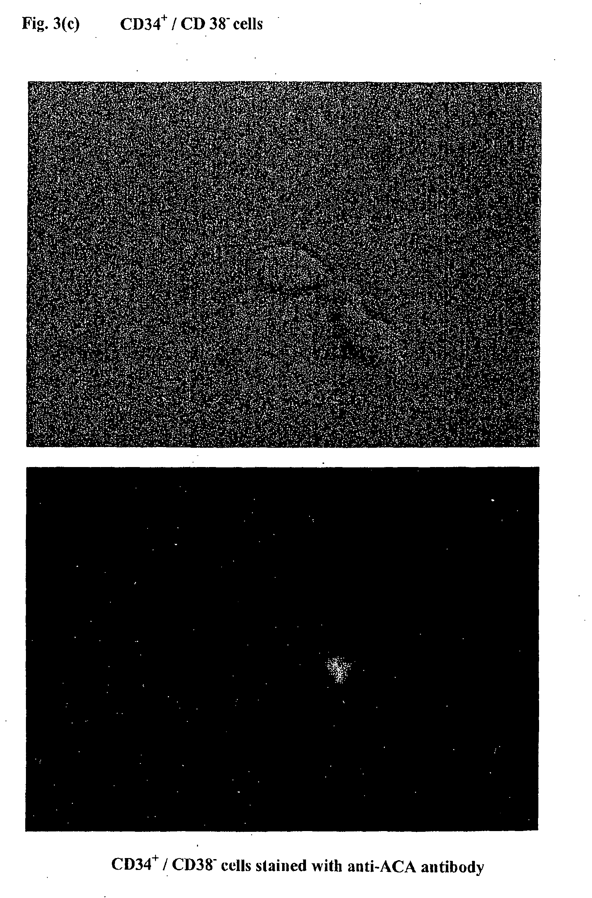

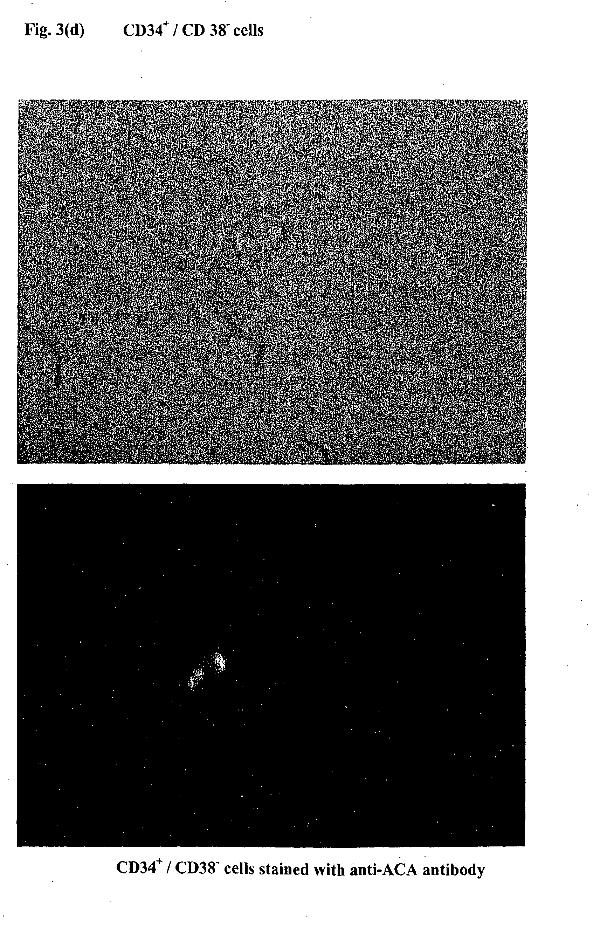

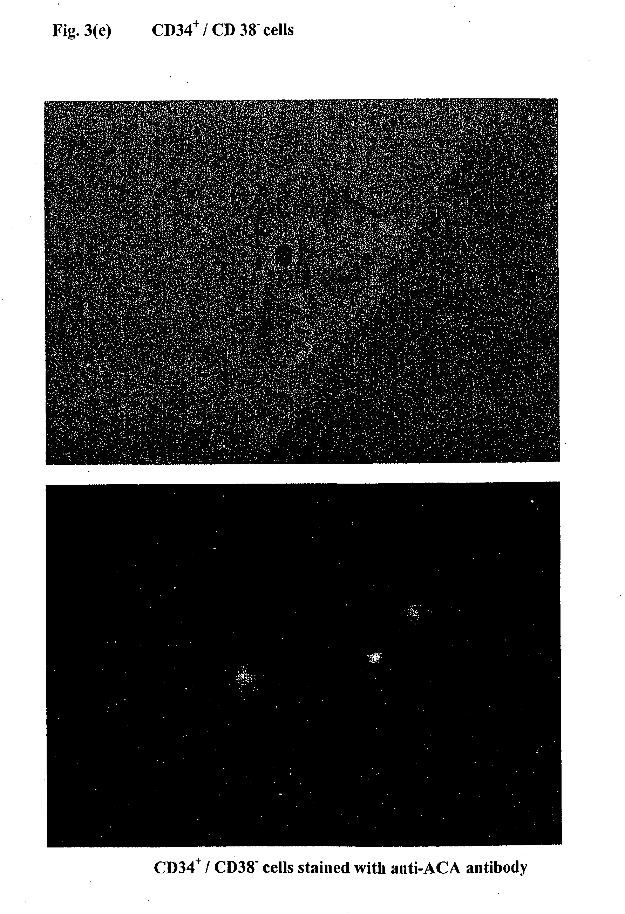

Mononuclear cells were isolated from umbilical cord blood, enriched for CD 34+ cells as described above, stained with CD38 PE and the CD34+/CD38- cells were than purified using fluorescence activated cell sorter. The isolated cells were placed in culture medium, incubated with ACA/goat anti-mouse Alexa Fluor 488 specific antibodies, and images acquired using an inverted fluorescence microscope (Figure 3).

### (4) Cross-linking (ligation) of CD34+/CD38+ cells with ACA specific antibody

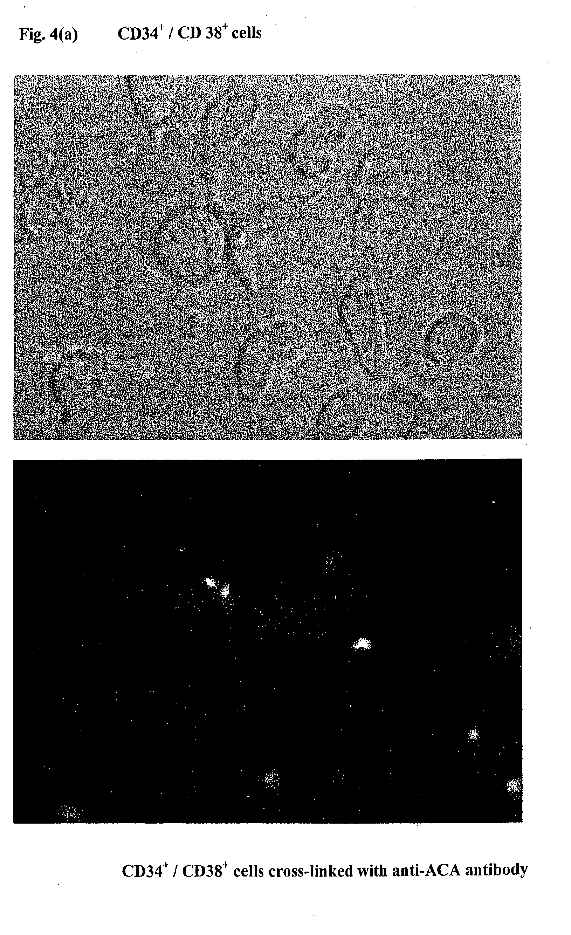

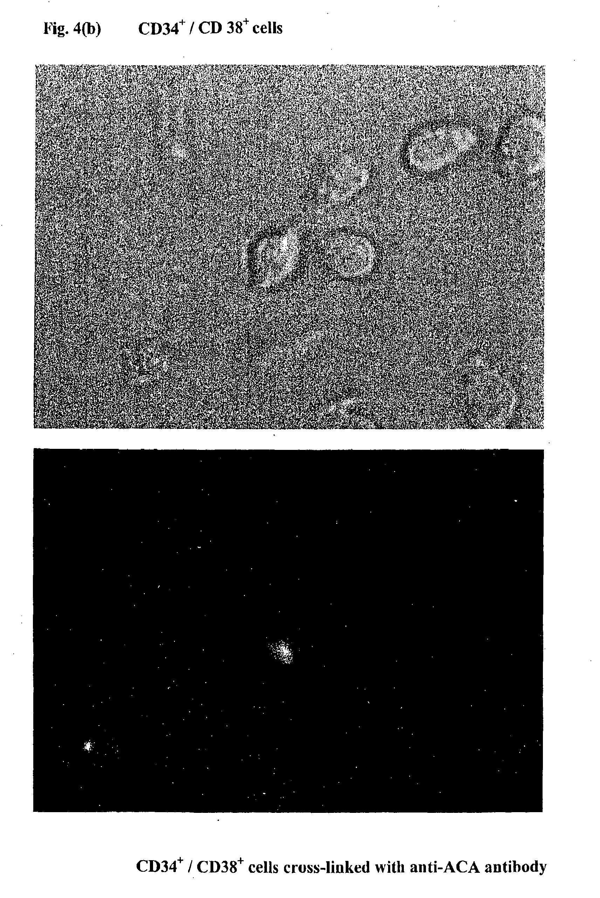

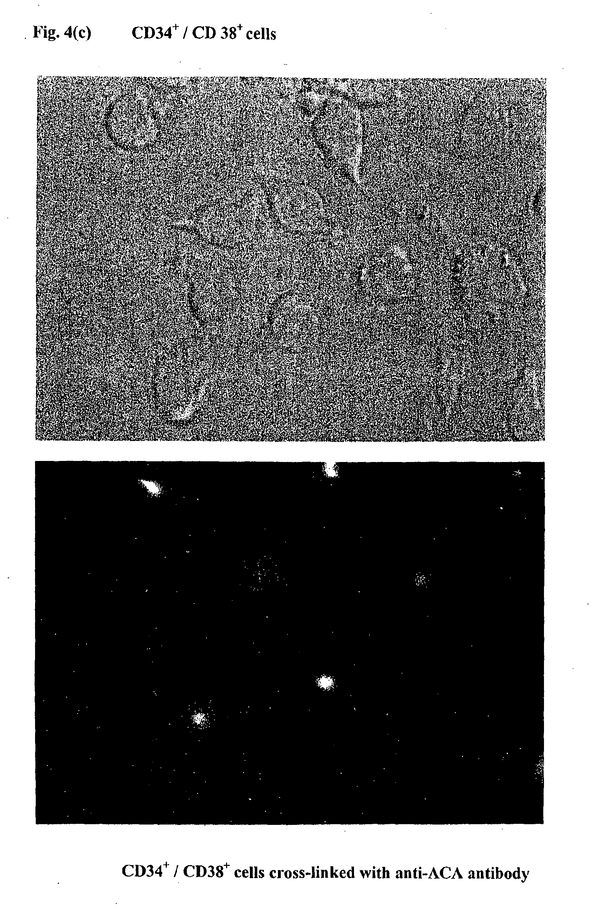

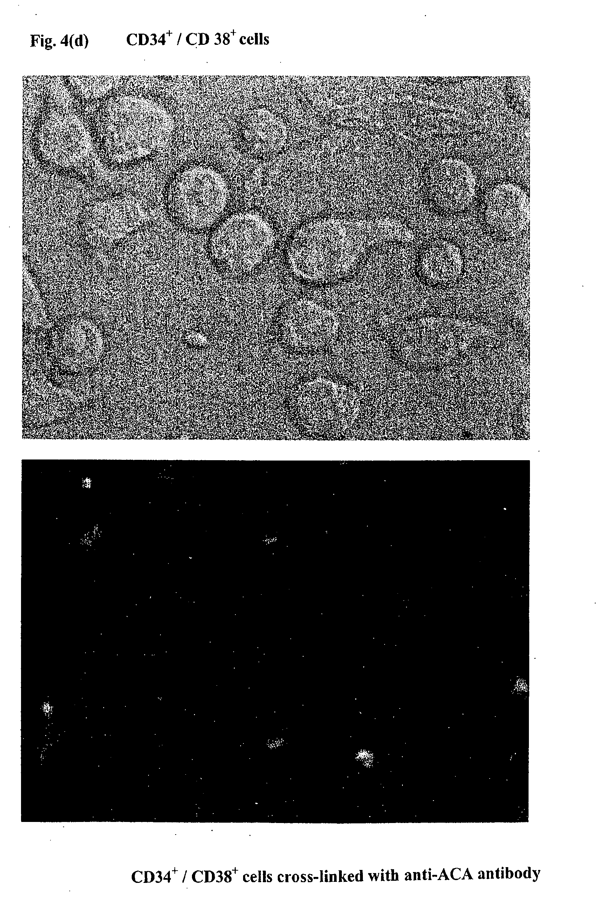

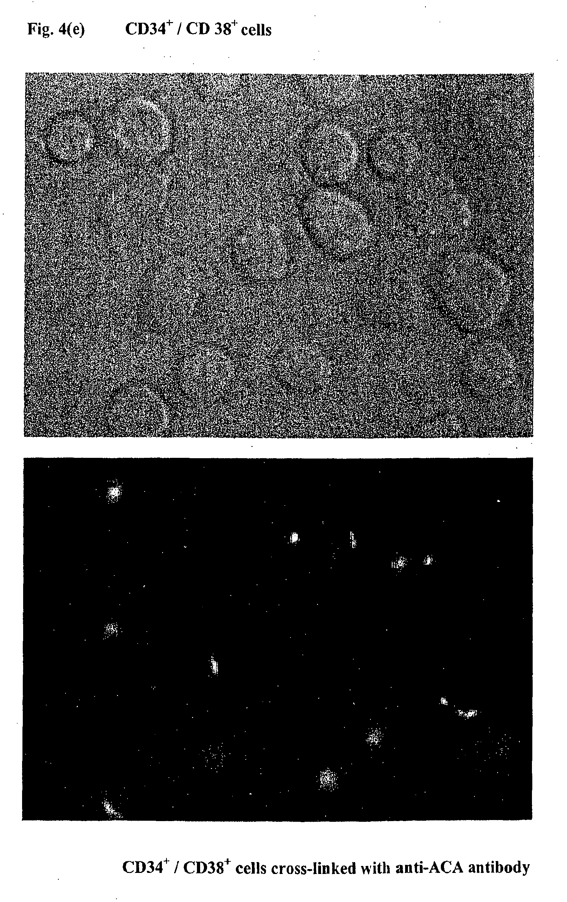

CD34+/ CD38+ cells obtained as already described, were incubated with ACA specific antibody, placed in culture plate with appropriate culture medium, and images acquired before and 24 hours after incubation with ACA specific antibody using inverted fluorescence microscope (Figure 4)

### (C) Conclusions

From the experiments described above the following conclusions can be drawn:
- ACA is expressed on both important hematopoietic stem (CD34+/38-), and progenitor (CD34+/38+) cell populations.
- CD34+/CD38+ cells are thought to represent a progenitor cell population with symmetrically dividing cells and limited capacity for (short term!) hematopoiesis. This population consists of round nonpolar cell which are morphologically terminally differentiated, (no further changes in morphology!) and show a uniform distribution of cell surface molecules. Interestingly, ACA is expressed on these cells in a specific microdomain in the membrane of these cells.
- CD34+/CD38- cells divide asymmetrically, have a high capacity for long-term multilineage hematopoietic engraftment, and are though to be highly enriched for stem cells. ACA is expressed on these cells exclusively in a polar (spindle!) part of the cell and constantly in the microdomain of the membrane where the stem cells have contact to each other.
- Cross-linking (ligation) of CD34+/CD38+ cells with an ACA specific antibody leads to complete transformation of this cell population. Round non-polar cells turned 24 hours later, to asymmetrically dividing cells, with typical "de novo" expression of ACA protein, clearly indicating signalling competence of ACA protein. ACA engagement promotes aggregation of lipid rafts which facilitates co-localisation of signalling proteins thereby potentiating protein tyrosine phosphorylation and subsequently activation of signal transducer proteins and proteins that activate transcription. Most importantly, direct stimulation of ACA with corresponding antibody is sufficient to keep the HSC in undifferentiated (pluripotential) state, and leads to massive expansion of hematopoietic stem cells in vitro.

Taken together, these data suggest that the ACA protein is a key molecule in the most important cellular process which maintain the pluripotential phenotype of human hematopoietic stem cell and can be exploited in clinical praxis without ethical baggage that accompanies embryonic stem cells.

## Claims

1. A method for obtaining or maintaining pluripotent non-embryonic stem cells, comprising contacting haematopoietic CD34+/CD38+ cells having glycosylphosphatidylinositol (GPI)-anchored glycoprotein ACA with an anti-GPI-ACA antibody.

2. Method according to claim 1, wherein said anti-GPI-ACA antibody is a monoclonal antibody.

3. Method according to claim 1 or 2, wherein the cells are from bone marrow, peripheral blood, cord blood, fat tissue, liver, muscle, skeletal myoblasts, cardiac myoblasts, endothelium or epithelium.

## Patentansprüche

1. Ein Verfahren zur Gewinnung und Erhaltung von pluripotenten nichtembryonalen Stammzellen, der den Kontakt vom hematopoietischen CD34+/CD38+ Zellen, die das Glycosylphosphatidylinositol-inositol (GPI)-verankerte Glycoprotein ACA exprimieren, mit anti-GPI-ACA -Antikörper, einschließt.

2. Eine Methode nach Patentanspruch 1, worin besagter anti-GPI-ACA-Antikörper ein monoklonaler Antikörper ist.

3. Eine Methode nach Patentanspruch 1 oder 2 worin die Zellen von Knochenmarkt, peripheren Blut, Nabelschnurblut, Fettgewebe, Leber, Muskel, Skelletmyoblasten, Herzmyoblasten, Endothelium oder Epithelium, entstammen.

## Revendications

1. Méthode pour l'obtention ou le maintien de cellules souches pluripotentielles non embryonnaires, comprenant le contact des cellules hématopoïétiques CD34+/CD38+ qui expriment ACA la glycoprotéine a ancrage glycosylphosphatidylinositol (GPI), avec an anticorps anti-GPI-ACA.

2. Méthode selon la revendication 1, où l'anticorps anti-GPI-ACA est un anticorps monoclonal.

3. Méthode selon les revendications 1 ou 2, où les cellules proviennent de la moelle osseuse, le sang périphérique, le sang de cordon ombilical, le tissu adipeux, le foie, les muscles, les myoblastes squelettiques, les myoblastes cardiaques, l'endothélium ou l'épithélium.
